# EUROPEAN PATENT SPECIFICATION

(11) **EP 1 863 394 B1**
(45) Date of publication and mention of the grant of the patent: **03.12.2008**
(21) Application number: 06719689.9
(22) Date of filing: 27.01.2006
(51) Int. Cl.: A61B 19/04, A41D 19/00

(54) **GLOVES WITH ENHANCED ANTI-CUFF-SLIP SURFACE**
HANDSCHUHE MIT ERHÖHTER STULPENRUTSCHFESTER OBERFLÄCHE
GANTS AVEC SURFACE AMELIOREE ANTI-GLISSEMENT DE MANCHETTE

(30) Priority: 31.03.2005 US 94959
(43) Date of publication of application: 12.12.2007
(73) Proprietor: KIMBERLY-CLARK WORLDWIDE, INC., Neenah WI 54956 (US)
(72) Inventor: WOON, Lin-sun, Alpharetta, Georgia 30005 (US); MODHA, Shantilal, H., Alpharetta, Georgia 30004 (US)
(74) Representative: Davies, Christopher Robert
(86) International application number: PCT/US2006/002945
(87) International publication number: WO 2006/107377

(56) References cited:
- WO-A-96/39055
- US-A1- 2005 044 609

## Description

### FIELD OF THE INVENTION

The present invention relates to elastomeric articles. In particular, the invention relates to certain work, medical, or surgical gloves that have a modified surface, which reduces cuff-slip down, for enhanced protection of wearers.

### BACKGROUND

Tight-fitting elastomeric articles, such as examination, medical, or surgical gloves, are usually made from either natural rubber or synthetic latex materials. Typically, such natural or synthetic latex materials are tacky and have a high coefficient of friction. This does not allow for easy introduction of a hand into the glove. Hence, some sort of surface treatment is required on the inner surface of the glove to facilitate donning, which may also involve lubricating the interior surface to enable ease of donning by a wearer because of undue clinging or friction between the surface of the article and that of the wear's skin. Traditionally, powdered lubricants have been applied to the inside surface of the glove to reduce friction between the skin and the glove. Unfortunately, the use of powdered lubricants may not be appropriate for specific situations, such as the case of surgical gloves. Specifically, if some of the powder escapes from the inside of the glove into the surgical environment, as for example if the glove is torn during the surgery, the powder may enter the surgical wound and cause further complications for the patient.

As a result, other solutions have been developed to aid in the donning of elastomeric gloves. Polymeric lubricant coatings have been developed to modify the interior surface of the gloves in an effort to provide a safe and effective donning for medical practitioners. To date, the surface modification coating that has had the best success and that is most accepted is a hydrogel polymeric coating.

Several kinds and ways of applying lubricating coatings, such as silicones or hydrogels, have been developed in the past; many with great success at alleviating the problem of donning. Coating the inner surface of elastomeric gloves with lubricant or donning layers, however, is believed to have unintentionally aggravated the tendency of the cuff regions of gloves to slip or roll down along the wrist during use. The problem is particularly burdensome with surgical gloves, in view of the critical nature of the surgeon's activities, and also in view of the danger that sterile field on the gloves may be compromised by attempts to re-roll the glove backup along the surgeon's arm. Surgical gloves currently on the market experience significant cuff slippage during use.

Attempts that solving the glove-cuff slippage problem have traditionally focused on changing the physical shape or design of the glove. For instance, some commercially available gloves are provided with relatively thick, circumferential or longitudinal bands, ribs, fluting, or beaded cuffs, in an attempt to minimize cuff roll-down. Alternatively, others have suggested applying an adhesive strip to inside of the cuff, and still others have suggested employing rather cumbersome straps and pegs to secure the cuff tightly to the wear's wrist and forearm. Some manufactures have remedied this issue by narrowing the diameter of gloves at the cuff opening or its immediate area, which has resulted in more constrictive force on the arms producing discomfort and often a compression band on the skin. Hence, these efforts have not been very successful.

A need exists for a better alternative that does not constrict the glove cuff, nor substantially change the gross physical configuration of the glove from designs which medical, surgical, or laboratory workers have become accustomed. The present invention satisfies this need and can provide such an alternative.

### SUMMARY OF THE INVENTION

The present invention pertains to the development of elastomeric articles, such as medical, surgical, or work gloves, that have a natural rubber latex or synthetic polymer latex substrate with a final interior surface that is differentiated into at least two major zones or regions, each with a different surface characteristic: At least one zone, a first region, of the elastomeric article is directed to achieving improved anti-slip or anti-roll-down characteristics when worn by a user against fabric or skin. To the extent that this first region of a glove has anti-slip properties, the surface of the glove is modified to have a higher coefficient of friction (COF) relative to the other, second region. In some embodiments, the higher COF may be achieved by either providing a more tacky surface in the first region or generating a slicker surface in the second region, each of which may involve the application or removal of a coating, such as of a hydrogel or other lubricating cover, to the respective zone. According to certain embodiments, the surface of the first region can be left substantially bare of any coating. That is, the elastomeric material (e.g., natural rubber or synthetic polymer) surface is exposed, or the region can be coated with a layer of adhesive or tackifying material (e.g., rubber cement). The difference in relative coefficient of friction helps prevent the glove from slipping down off of a sleeve of a surgical gown or other garment surface. Hence, the anti-slip first region preferably should be situated at or near the cuff portion of the glove. The difference in relative coefficients of friction between the two regions or zones of the inner, wearer-contacting surface can be expresses as a ratio in a range of at least about 1:1.2, up to about 4.5, more typically about 1:1.3 to about 1:3.8, preferably, about 1:1.4 to about 1:3.35. The first region, depending on the physical dimensions or size of the glove, can cover a distance or width that extends inwardly, from the terminal edge of the glove cuff, about 1 inch (2 cm) to about 5 or 6 inches (~12-13cm), and around laterally over a surface area that encompasses the entire periphery of the cuff area.

Just as the first region of the glove, absent a slick coating, will exhibit a relatively more tacky surface, it is envisioned that the second region of the glove includes a donning layer, such as a hydrogel or lubricating silicone layer, which will exhibit a low friction, sleek surface. This may be accomplish in the second region through application of a variety of different kinds of donning coatings that, for example, may either completely or partially cover the surface to assist donning requirements.

In another aspect, the present invention also pertains to an online method of fabricating an elastomeric article having a surface with differentiated coefficients of friction. That is, one can create the different surfaces while the elastomeric article is still on its mould, according to a dipped-goods technique. The method encompasses: forming or providing a natural or synthetic rubber latex substrate; subjecting the latex substrate to a first water leach; applying an aqueous AlSO₄-containing solution to a surface of the latex substrate; applying a donning coating over a part of the AlSO₄-coated surface, such that a region of about at least 1 inch (∼2.5 cm) from a terminal edge of the latex substrate remains uncoated with the donning coating. After the donning coating is dried and cured, second layer of AlSO₄ is applied over the donning coating, followed by at least a second water leach. This application seals the donning coating, and prepared the coated latex substrate surface for exposure to a dilute solution of an oxidizing agent under alkaline conditions of about pH 9-11. The oxidizing agent can be a hypochlorite (e.g., NaOCl) solution, which is adapted to impart a sleek or smooth finish to the donning coating.

Additional features and advantageous of the present invention will be revealed in the following detailed description. Both the foregoing summary and the following detailed description and examples are merely representative of the invention, and are intended to provide an overview for understanding the invention as claimed.

### BRIEF DESCRIPTION OF FIGURES

Figure 1 is a representation of a glove according to the present invention, as worn on the hand.
Figure 2 shows the cuff region of the glove depicted in Figure 1, partially rolled back over the hand portion of the glove.
Figure 3 is a stylized depiction of the glove depicted in Figures 1 and 2, showing differentiated surface regions or zone on the donning side of the glove. In one zone, near the cuff, the article has an anti-slip surface that has a higher relative coefficient of friction than the other zone.

### DETAILED DESCRIPTION OF THE INVENTION

### Section I - Article

In general the present invention is directed to an elastomeric article. As used herein, the term "elastomeric article" refers to an article formed predominantly from an elastic or elastomeric polymer latex. An "elastomeric polymer" refers to a polymeric or rubber latex material that is capable of being stretched or expanded, and upon release of the stretching or expanding force, will return to substantially (e.g., ± 5%) its previous size and configuration. An article according to the present invention, for example, may include clean-room, surgical, work, and/or industrial gloves, that general exhibit a tendency to slip down over time when worn or used. Hence, the present invention is adapted to combat this problem, by providing, in part, a glove with at least two zones on its inner surface with differentiated surface characteristics. One of the zones is adapted to prevent slippage of the article when in contact with either human skin, or a fabric material by engaging with the skin or fabric material.

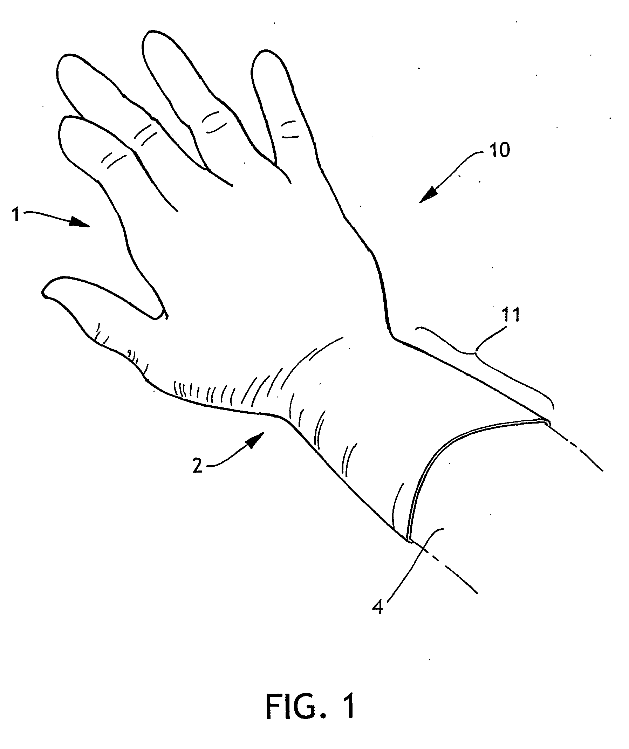

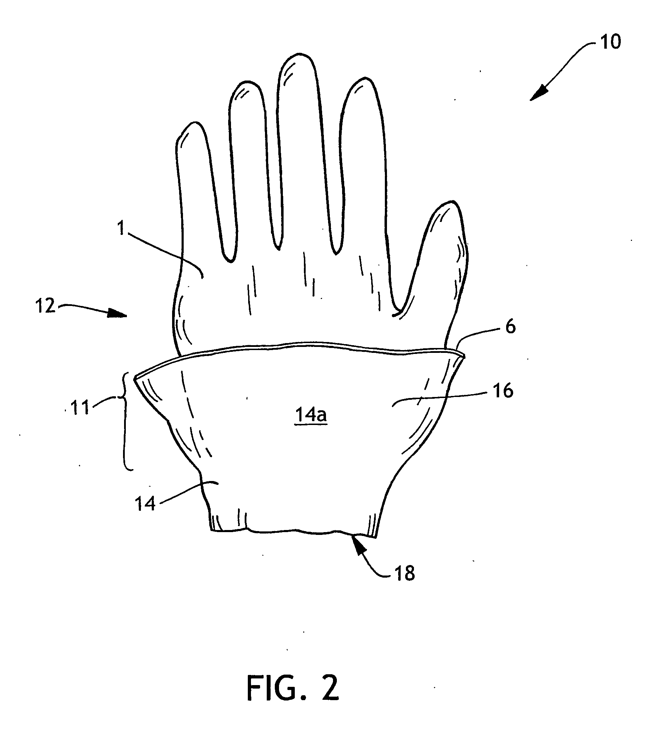

Figure 1 depicts a glove 10 that has a flexible, substrate body formed largely from an elastic, natural or synthetic latex material. The substrate body has a first surface and a second surface. For purposes of description, the first surface refers to the inner surface of the glove substrate body which when worn by a user in use is proximal or near to the body or skin of the wearer. The second surface refers to the outer surface of the article, which is remote from the body and skin of the wearer. The inner first surface is differentiated into at least a first and a second zone or region. Figure 2 shows a partially inverted view of the glove 10 of Figure 1, according to the present invention, which includes an elastomeric substrate body 12 with an donning side inner surface 14 made up of at least a first 16 and a second region 18. Each zone or region has a coefficient of friction, such that the coefficient of friction for the first zone or region 16 is greater than the coefficient for the second zone or region 18, according to a ratio in a range from about 1.2:1 to about 4.5:1. More typically, an average coefficient of friction ratio of the first zone to the second zone is about 1.3:1 to about 3.5 or 3.8:1. Desirably, the ratio is about 1.4:1 to about 2.75:1. More desirably, the coefficient of friction ratio is about 1.5:1 to about 2.5:1.

In the present invention, as a general observation an inner surface of a glove that has a higher coefficient of friction is more desirable because of friction to retain the glove against the material of a garment. Yet, this observation has caveats that were surprisingly unexpected. Gloves, especially surgeon's gloves, should not restrict the natural movement and flex of a wearer's hands and wrist. Hence, the glove should not be too stiff and the coefficient of friction ratio should not be too large. In our experience, generally, a COF ratio that exceeds about 1:4.0 or 4.1 can overly restrict the ability of the user to freely move and flex the user's hands and wrists. Further, a high COF will hinder the ability of the wearer to easily don the glove, especially under damp-skin conditions. The wear's hand will catch against the high friction surface of the cuff region. To strike a balance between maintaining a natural feel, without binding the movement of hands and wrists, and better donning, while preventing the glove-cuff from slipping down, an optimized range for the COF ratio is between about 1:1.4 up to about 1:3.4 or 3.5, preferably about 1:2.0-3.0.

As depicted in Figure 2, the first region 16 is at least part of the inner surface 14 at or about a cuff portion 11 of the glove, and is adapted to exhibit a tendency to retard the tendency of the glove cuff slipping down from a wearer's wrist 2 or forearm 4 when in use. The glove may have a bead 6 at the terminal edge of the cuff, which further retards slippage. The first region 16, according to an embodiment, is absent the donning coat layer. The surface of the first region 16 may be either a bare, uncoated elastomeric material; or alternatively, may be covered with at least a layer of a tackifying material. In other words, the surface characteristics of the first zone can be modified to create a higher relative coefficient of friction than the second zone, by means of either taking away or adding coating layers. The inner surface at the first region 14a can contact an article of protective clothing, such as a surgical gown, coverall, smock, or other garment, and engage with the garment because of the first region's relatively higher coefficient of friction. The higher coefficient of friction can, in some embodiments, be derived from a greater tackiness on the first zone. Since the second region 18 forms at least part of a surface that contacts the wearer's skin, typically the hand 1 and wrist 2 portions of the glove, the second region 18 can be either substantially larger or about equal in surface area than the first region 16, and include at least a partial coating of a donning material, such as a hydrogel-based polymer. Alternate donning layer materials may include or be selected from, for example, polyurethanes, polymers from the poly-acrylate family, polyvinyl alcohol (PVA) condensation products, poly(styrene-butadiene-styrene) (SBS), or poly(styrene-ethylene-butadiene-styrene) (SEBS).

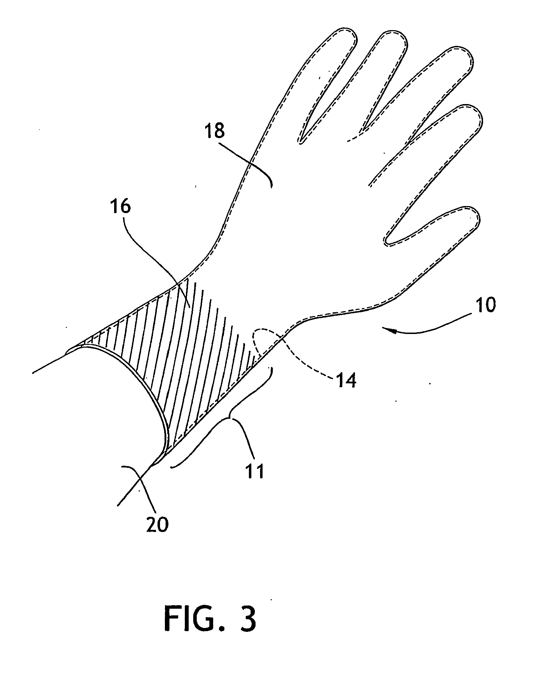

According to the present invention, the donning coat covers only partially the interior surface of a glove. That is, in an embodiment, a region of one to several inches (e.g., 1-5 or 6 inches) at or about the cuff portion of a glove is left free of a hydrogel coating, which covers the hand-donning portion around and/or below the wrist to the finger tips. The area of rubber film without hydrogel coating provides a tacky surface to adhere to gown fabric and this prevents the glove from slipping under normal use by the wearer. The tacky surface may also provide a seal against fluids draining under and into the glove, and afford more protection to the user. This is more easily illustrated, as shown in Figure 3, with a glove 10 that is prepared just before being stripped from its mould 20 and inverted, with what will become in the inner surface 14 on the outside, away from the mould.

### Section II - Process

In another aspect, the present invention pertains in part to fabrication of elastomeric gloves according to either a post formation tumbling process or an online process for increasing the relative difference in coefficient of friction of the cuff portion and hand-donning portion of an elastomeric glove. The term "online process" refers to a mechanically automatable or automation-friendly course of production, in which gloves do not need to be removed from their moulds during the manufacture and treatment steps. An elastic glove, is customarily fabricated by dipping processes, which, typically, involve the use of a hand-shaped mould, also known as a former. According to either approach of the method, a hand-shaped former, is first dipped in a coagulant solution bath to coat the mould. The mould can be composed of a variety of materials, such as either metal or ceramics, and can have a roughened or textured surface, or a smooth surface. The coagulant is allowed to dry before dipping the mould into an emulsion of a natural rubber or synthetic polymer latex, which is applied to cover the mould. The mould dwells in the latex for a short time period, about 1-5 minutes, to deposit a film layer of predetermined thickness. The latex film is allowed to partially dry. A bead may be formed at the terminal edge of the latex film. The latex film is soaked in hot water at about 40-80°C to leach out impurities. The leached latex film, while still on the former, is then either sprayed with or dipped into a dilute solution of AlSO₄ (e.g., ActiveBond^{™} by Delta Polymer, Inc.) with a total solids content of about 0.2-3.5%, desirably about 2-3%, to apply a uniform layer to the latex substrate surface. Afterwards, the glove is dried at a temperature in a range of about 60-150°C, preferably about 100-140°C.

In some embodiments, a hydrogel-based donning coat is applied to the dried latex film to cover the portion of the glove that will be in contact with the hand. AlSO₄, it is believed, acts as a gelling coagulant for the hydrogel polymer and to enhance its attachment to the rubber substrate of the glove..Examples of possible suitable hydrogel polymer coatings are described in various references, such as U.S. Patent No. 4,548,844 and 4,575,476. both to Podell et al., and U.S. Patent No. 4,499,154 to James et al*.* all disclose gloves with a continuous inner layer of such hydrogel polymers; or U.S. Patent Applications Nos. 10/978343, and 11/023743. The hydrogel solution can be about 1-5% total solids contect (TSC). The hydrogel layer is allowed only to cover part of the original latex surface. The hydrogel dip is maintained at a level that is at least 1-2 inches below the upper terminal edge or bead of the glove cuff. Preferably, the dip-level is lower by about 3-6 inches, depending on the overall length and configuration of the glove. That is, a glove with a longer cuff region desirably will have a proportionately larger area that is left uncoated with the hydrogel polymer. After the hydrogel coating is applied to the glove surface, a second layer of AlSO₄ is applied over the hydrogel layer. It is believed that this second layer provides an acidic surface to the hydrogel layer.

To control self-blocking or sticking of the latex substrate to itself and to facilitate stripping of the glove from its mould, one applies a layer or coating of a fine powdered release agent, such as calcium or magnesium carbonate (CaCO₃, MgCO₃)), magnesium or zinc stearate (Mg(C₁₈H₃₅O₂)₂, Zn(C₁₈H₃₅O₂)₂), from a slurry dip over the entire glove to serve as an inorganic release agent.

Another way to obtain differentiation on a rubber surface (i.e., to achieve a two-zone effect without hydrogel coating) involves treating only part of the inner surface. That is, to partially chlorinate part of the donning side, and the area of the inner surface that is left un-chlorinated is treated with a silicone lubricant to generate a higher COF than the chlorinated surface.

In contrast to current, conventional processes that use gaseous chlorine at acidic pH levels to halogenate the hydrogel layer, an attribute of the present invention involves exposing the gloves, while still on the former, to bath of an alkaline solution of an oxidizing agent, such as sodium hypochlorite bleach. The solution will have a pH ≥8 or 9. It is believed that the hypochlorite works as a dilute chlorinating agent for the hydrogel coating and uncoated rubber surface, and imparts a sleek finish to the hydrogel coating for improved donning properties. In other words, sodium hypochlorite is used as a processing aid to create a smoother and sleeker hydrogel coating for easier donning characteristics. This process step involves no chlorine gas to enhance the relative smoothness of the coated surface for better donning characteristics and relatively lower coefficient of friction. Further, when processing online, with the donning layer exposed to out side, the sodium hypochlorite modifies only the donning side and one can avoid exposing the gripping region or outer surface of the glove. In other words, as the glove is still on the former, one can easily control the area and size of glove surface that is exposed to chlorine by controlling to what level or how far the glove substrate is submerged into the bleach solution. The hypochlorite coated area can be coterminous with the hydrogel coated region Further the present process will provide precise control at level of chlorination needed to facilitate the donning requiring. The present online process will improve, batch to batch, the consistency of the halogenation process compared to current process using gas and off line process. After at least a second aqueous leaching, which removes most of the second AlSO₄ application and powdered release agents, the glove is dried and cured at temperatures in a range of about 90-150°C for about 20-40 minutes.

Afterwards, the method may further comprising stripping the latex substrate from a mould, inverting the latex substrate, and applying a lubricating layer over the outside of the glove in an off-line processing. A lubricating layer formed from a silicone, such as a polydimethyl siloxane emulsion, can be applied at about 0.1-1.3 or 1.5 weight percent in an aqueous tumble inversion-rinse. Desirably, the silicone emulsion is applied to the glove in an amount of about 0.2-0.25% by vol.(- 0.080-0.150% by wt.). In some embodiments, the silicone emulsion may have a total solids content (TSC) of not less than about 20% and up to about 60%. An example of a desirable silicone emulsion is a pre-emulsified formulation known as DC 365 (35% TSC), available commercially from Dow Corning Corporation (Midland, Michigan). DC 365 is believed to contain about 40-70 mass % water, about 30-60 mass % methyl-modified polydimethylsiloxane, and about 1-5 mass % octylphenoxy polyethoxy ethanol. Other examples of pre-emulsified silicone formulations that may be suitable for use with the present invention are available commercially from GE silicones (Waterford, New York) under the trade names SM 2140 (50% TSC), SM 2169, and AF-60. SM 2140 is believed to contain about 30-60 mass % water, about 30-60 mass % amino-modified polydimethylsiloxane, about 1-5% mass %trimethyl-4-nonyloxypolyethyleneoxy ethanol, and minor percentages of acetaldehyde, formaldehyde, and 1,4 dioxane. SM 2169 is believed to contain about 30-60 mass % water, about 60-80 mass % polydimethylsiloxane, about 1-5 mass % polyoxyethylene lauryl ether, and a small amount of formaldehyde. AF-60 is believed to contain polydimethylsiloxane, acetylaldehyde, and small percentages of emulsifiers. If desired, these pre-emulsified silicones may be diluted with water or other solvents prior to use.

Silicone emulsions function as a lubricant on polymer-coated surfaces, but on surfaces not coated with polymer, the silicone emulsion enhances overall tackiness. Hence, the polymer-coated sections of the glove become even more slick, which is good for donning, while the areas of the substrate where there is no polymer coating the silicone emulsion provides a desired grip level, such that the inner surface of the glove will adhere to a gown material when worn. The silicone emulsion is applied to both hydrogel-coated and non-coated areas of the glove substrate.

After the glove has been stripped from its mould, the latex substrate may be further subject to a series of at least two consecutive rinses with heated water (~27°C or 30-40-88°C). In so-called backend processing operations, the glove is treated with an effective amount of chlorine to halogenated the gripping side. A chlorine-containing aqueous solution (at - 30 - 75 ppm) can be used to process the grip side of the glove, followed with wash in a dilute hydrochloric acid (HCl) solution (about 0.1-0.85% by volume, desirable about 0.2 or 0.25-0.37%) to remove any release agent power that may remain after the water rinses. HCl dissolves the CaCO₃ to produce a salt, which is then washed away by water to produce a powder-free product.

Alternatively, a batch of gloves can be tumbled in softened or deionized water as a pre-rinse. We envision, according to another aspect of the invention, a one-step treatment involving exposing the gloves to a combined mixture containing HCl+ chlorine + silicone emulsion in either a bath or atomizing stray in a tumble process. The combined HCl-chlorine-silicone treatment can produce in one step several different surface effects, which ordinarily would require several different process steps. The silicone attaches to the non-polymer coated surface and the chlorine attaches to the partial polymer-coated areas of the glove, and the HCl helps remove excess CaCO₃ powder that may be present. Then, the gloves are again rinsed two times in softened water, followed by rinse four times in deionized water. The batch of gloves is then dried at about 70°C to about 80°C.

The chlorine and HCl treatment is believed to impart a smooth and sleek finish, enhancing the donning properties of the hydrogel-coated rubber surface. Generally, the higher the percentage chlorine content, the slicker the finished rubber surface becomes with an attending reduction in COF. Subsequently, one may further immerse and tumble rinse the glove for an additional third or fourth time to remove either powder residues or latex proteins in natural rubber-based gloves. Further, one may rinse up to an additional two more times in deionized water baths. Finally, another layer of silicone emulsion (e.g., DC 365) is applied in aqueous treatment via a tumble inversion-rinse, and the glove is dried.

When comparing the two versions of the present process treatment, the online approach tends to produce more consistent results, hence it is a more cost-effective and desirable process than the tumble approach.

### Examples

### Example 1.

According to the online process the hand-shaped former is dipped in a coagulant bath to coat the mould. A natural or synthetic rubber latex is applied by dipping into a bath and partial dried on the mould. A bead may be formed at the cuff. While the latex substrate is on the former, the glove is subject to a first water leach. An AlSO₄ (e.g., Active Bond^{™} solution (~1%) is applied by either spraying or dipping of the glove into an aqueous bath for about 1-3 minutes, over the entire surface of the glove, up to and including the glove cuff. AlSO₄ functions as a primer that helps enhance subsequent surface modifications. A donning coat, such as a hydrogel or polyurethane layer, is applied over part of the AlSO₄-coated surface. The AlSO₄ on the surface of the hydrogel layer is unreacted with the hydrogel. Typically, an area with a width of about 1 or 2 inches to up to about 5 or 7 inches (~2.5 -13 or 16 cm), down from the end of the glove cuff or bead, if present, is left uncoated with the donning coat. Preferably, the region left uncoated is about 1-2 inches (~2.5 - 5 cm). The donning coated is dried and cured. The glove is subject to a second water leach and dried. The entire glove can then be either immersed in a bath or sprayed with a hypochlorite (NaOCl) solution and dried. Online treatment with NaOCl can simplify the post processing, by removing the chlorination process conventionally performed in tumblers. The water rinse removes the AlSO₄ treatment, hence the AlSO₄ does not react with the hypochlorite.

According to the present invention, chlorination process takes place under alkaline conditions of pH ∼9-10 or 11, which are gentler and more conducive for online fabrication. Under acidic conditions, the hydrochlorous acid (HOCI) will tend to dissociate and release chlorine gas, which can be quite caustic and harsh, hence not useful in an online process. In our process, it is believed that the NaOCl affects the hydrogel or polyurethane coating only to make it slick for donning. This is done in a dip tank with the online process. The percentage chlorine is present in a range of about 0.2-1.0%. Typically, chlorine is at about 0.3% (3000 ppm of Cl₂). The available amount of chlorine in NaOCl is about 6%. According to some embodiments, the effective amount of chlorine is about 6% of 0.3%, or about 0.018%.

The hydrogel coating can be slickened with exposure to an oxidizing agent, such as a solution of sodium hypochlorate, which is desirably applied co-terminus with the hydrogel donning coated region. Afterwards, the cured glove is soaked in hot water at about 40-80°C for about 0.5 to 3 or 4 minutes. After drying for a few minutes 3-6 minutes, the glove can be dipped in a slurry of calcium carbonate (2-5%) suspended in water. When the slurry is dried, the glove can be removed from the ceramic mould. The stripping and inversion of the glove will make the partial-hydrogel-coated surface to become the interior surface of the glove to facilitate donning.

A suitable silicone emulsion, such as commercially available formulations from Dow Corning (DC 365) or General Electric (AF60) can be applied to increase the coefficient of friction on the uncoated latex surface.

### Example 2.

In an alternate tumbling process, a surgical glove made from an elastic latex can be manufactured by dipping the former into a coagulant solution. As with the previously described steps, a former is coated with a layer of either natural rubber or synthetic latex and allowed to partial dry. A bead may be formed at the end of the latex substrate. A first water leach is applied, followed with a coating of AlSO₄ over the entire exposed surface of the latex substrate on the former. A partial hydrogel coating is applied only over the hand and wrist regions of the mould, dried and cured. A band or region of latex is left uncoated. This band can have a width of about 1-6 inches, preferably about 1-2 inches (2.5 - 5 cm), from the terminal edge or bead, if present. The glove is subjected to a second water leach and dried. The entire glove is then dipped into a CaCO₃ slurry, and dried. Afterwards, the glove is stripped from the mould and inverted. The subsequent process steps and chemical treatments are conducted in a tumbling drum, such as in a washing machine. Another coating of AlSO₄ is applied in a tumble inversion-rinse over the entire glove and dried. A first treatment with silicone emulsion in water is then applied. After at least two rinses in water, the glove is subject to a rinse in HCL solution, followed by another at least two rinses in softened water, and two more rinses in deionized water. Finally, a second treatment with silicone emulsion is applied and the glove is dried.

### Section III - Coefficient of Friction

It is believed that the modification of the inner surface of a glove, according to the present invention can greatly reduce the tendency for elastomeric gloves to slip when worn, especially when in contact with an article of protective clothing. A portion of the glove, not coated with donning layer, but a tacky surface engages with the material of the protective clothing. As such, one creates a good glove-gown interface region. In other words, due to the combination the silicone emulsion over the latex substrate which generates a higher COF at the cuff region than over the hand-donning region of the glove. The cuff sticks to gown surface and reduces slip down.

Two different tests were utilized in the determination of the coefficient of friction:
(1) Classical Inclined Plane and Weight Method: This test measures coefficient of friction via a determination of the angle at which the test sample, weighted down in a standard manner, slides down an inclined plane. The test is generally described, in Principles of Physics, by J. B. Marion et al., chapter 7-1, Saunders College Publishing, New York, N.Y., 1984.
(2) Kawabata Method: This test is described in Standardization Analysis of Hand Evaluation, by Sueo Kawabata; July, 1980, 2nd Edition, pp 31-35, 48-50. As compared to the Classical Method, the Kawabata test is more reliable for measuring the coefficient of friction on rough surfaces or surfaces with small areas. In this method, the test material is moved from left to right while a contacting element (of specific dimensions, and under constant force) touches the surface of the material. A transducer connected to the detector is used to measure frictional force as the test material is moved.

Tests using the Classical Method were repeated five times on each sample to determine mean value. Tests using the Kawabata method were repeated six times: two times for each sample. The Kawabata COF data for two sizes (i.e., 6.5 & 7.5 inches) and 3 measurements of each size for each product sample were taken. The coefficient of friction is determined on the cuff and palm areas of the samples. In the test zone A is defined as the surface of the glove within 1.25 to 2 inches from the cuff edge or bead, and zone B is the surface of the donning areas of the glove, in particular the palm region. Table 1 summaries the mean average values of the Kawabata test results.

**TABLE 1.**

| Sample No. | zone A | zone B | Ratio (C.O.F. of zone A v. zone B) |
|---|---|---|---|
| 1 | 4.01 | 0.93 | 4.3: 1.0 |
| 2 | 1.67 | 0.72 | 2.3: 1.0 |
| 3 | 0.74 | 0.52 | 1.4: 1.0 |
| 4 | 2.38 | 0.68 | 3.5: 1.0 |
| 5 | 1.54 | 0.55 | 2.8: 1.0 |
| 6 | 0.91 | 0.57 | 1.6: 1.0 |
| 7 | 2.43 | 0.64 | 3.8: 1.0 |
| 8 | 1.85 | 0.75 | 2.5: 1.0 |
| 9 | 1.34 | 0.61 | 2.2: 1.0 |
| 10 | 2.36 | 0.76 | 3.1: 1.0 |
| 11 | 3.16 | 0.79 | 4,0: 1.0 |
| 12 | 0.73 | 0.52 | 1.4: 1.0 |

The above results clearly demonstrate that gloves produced according to the present processing method exhibit an enhanced friction characteristics as compared to the untreated counterparts. The Kawabata test demonstrated enhanced friction for samples 1, 4, 5, 7, 10, and 11. Since low friction is the fundamental cause of slippage at the cuff-sleeve interface, it appears clear that the increases in the relative COF set forth in Table 1 will provide a reduction in slippage without associated discomfort, such as over-constriction on the forearm, which tend to be associated with anti-slippage schemes that employ compression.

Table 1 also demonstrates that in some instances, relatively low levels of the treatment agent (e.g., samples 3, 12) were effective in significantly reducing the COF. As mentioned above, the use of lower levels is often desirable in view of costs, and in view of the tendency for gloves with high levels of the tackifying agent to sometimes become stiff. As mentioned before for gloves, COF ratios of over about 4.0:1 tend to hinder donning, and overly tether the glove cuff to the user's forearm area and can restrict the free movement or flex of the hands during use. This, however, is not to disclaim the potential advantages of a high COF differential for other applications which may appreciate a surface with a higher coefficient or more tacky grip.

The present invention can be adapted to any base rubber latex material formulation or dipping process. Hence, a variety of products may employ the present technique. Such products may include medical exam, surgical, or work gloves, condoms, as well as any article that may require at least a two zone differentiated surface having a contrast in coefficient of friction, such as for better wearability.

The present invention has been described both in general and in detail by way of examples. Persons skilled in the art will understand that the invention is not limited necessarily to the specific embodiments disclosed. Modifications and variations may be made without departing from the scope of the invention as defined by the following claims or their equivalents, including equivalent components presently known, or to be developed, which may be used within the scope of the present invention. Hence, unless changes otherwise depart from the scope of the invention, the changes should be construed as being included herein.

## Claims

1. A flexible article comprising: a substrate body (12) formed largely from an elastomeric material, said substrate body (12) having a first surface (14) and a second surface; said first surface is differentiated into at least a first and a second zone (16, 18), each zone (16, 18) having a coefficient of friction, such that the coefficient of friction for said first zone (16) is greater than the coefficient for said second zone (18), according to an average ratio in a range from about 1.2:1 to about 4.5:1 said first zone (16) is absent a donning layer, and said second zone (18) has said donning layer.

2. The elastomeric article according to claim 1, wherein said average coefficient of friction ratio is about 1.4:1 to about 3.8:1 for said first zone (16) to said second zone (18).

3. The elastomeric article according to claim 1, wherein said first zone (16) has a surface that is a bare elastomeric material or coated with a tackifying material.

4. The elastomeric article according any one of the preceding claims, wherein said first region (16) is part of a surface adapted to prevent slippage of said article when in contact with either human skin, or a fabric material.

5. The elastomeric article according to any one of the preceding claims, wherein said article is a glove (10).

6. The elastomeric article according to any one of the preceding claim, wherein said first region (16) forms part of a glove cuff, and said second region (18) forms at least part of a hand-donning portion of said glove (10).

7. The elastomeric article according to any one of the preceding claims, wherein said second region (18) is either substantially larger or about equal in surface area than said first region (16), and having at least a partially coating of a donning material layer.

8. The elastomeric according to any one of the preceding claims, wherein said inner surface (12) is subject to an oxidizing agent.

9. The glove according to any one of the preceding claims, wherein said inner surface (12) is subject to a partial halogenation, such that said first region is un-chlorinated and said second regions is chlorinated.

10. A method of fabricating an elastomeric article having a surface with at least two regions with differentiated coefficients of friction, the method comprises: providing a natural rubber or synthetic polymer latex substrate on a mould; subjecting said latex substrate to a first water leach; applying an aqueous AlSO₄-containing solution to a surface of said latex substrate; applying a donning coating over a part of said AlSO₄-coated surface, leaving a zone of about at least 1 inch (-2.2 cm) from a terminal edge of said latex substrate uncoated with said donning coating; drying and curing said donning coating; subjecting said latex substrate to a second water leach; applying a second layer of AlSO₄ over said donning coating; and exposing said latex substrate to a dilute solution of an oxidizing agent under alkaline conditions of about pH ≥ 8.

11. The method according to claim 10, wherein said pH is about 9-11.

12. The method according to claim 10, wherein said donning coating includes a hydrogel material.

13. The method according to claim 10, wherein said oxidizing agent is a hypochlorite (NaOCl) solution.

14. The method according to claim 10, further comprising stripping said latex substrate from said mould, inverting said latex substrate, and applying a lubricating layer formed from a silicone emulsion.

15. The method according to claim 10, wherein said silicone emulsion imparts either a tackifying effect to said zone absent said donning coating or a slickening effect to said donning coating.

16. The method according to claim 10, further comprising treating said latex substrate to a first and second consecutive rinse with water, and a chlorine-containing water at ∼ 30 - 75 ppm vol., and a dilute hydrochloric (HCl) solution of about 0.1-0.85% by volume to remove any remaining powdered release agent after said first and second water rinses.

17. The method according to claim 10, further comprising subjecting said latex substrate to a medium including a combination of HCl + chlorine + silicone emulsion in one process step to achieve tacky a surface on non-donning-material coated surface and a sleek surface on donning material-coated surface.

18. The method according to claim 10, further comprising applying another layer of silicone emulsion treatment.

19. The method according to claim 10, wherein said elastomeric article has at least a first and a second zone on an inner surface, in which said first zone has a relative higher coefficient of friction, according to an average ratio in the range of about 1.4:1 to about 3.5:1.

20. The method according to claim 19, wherein said average ratio of coefficient of friction between first and second regions is about 1.8:1 to about 3.0:1.

## Patentansprüche

1. Flexibler Artikel, umfassend: einen Substratkörper (12), der im Wesentlichen aus einem elastomeren Material gebildet ist, wobei der Substratkörper (12) eine erste Oberfläche (14) und eine zweite Oberfläche aufweist; wobei die erste Oberfläche in wenigstens eine erste und eine zweite Zone (16, 18) unterteilt ist, wobei jede Zone (16, 18) einen Reibungskoeffizienten aufweist, so dass der Reibungskoeffizient für die erste Zone (16) größer ist als der Koeffizient für die zweite Zone (18), entsprechend einem mittleren Verhältnis in einem Bereich von ungefähr 1,2:1 bis ungefähr 4,5:1, wobei die erste Zone (16) keine Anziehschicht aufweist und die zweite Zone (18) diese Anziehschicht aufweist.

2. Elastomerer Artikel nach Anspruch 1, wobei das mittlere Reibungskoeffizientenverhältnis ungefähr 1,4:1 bis ungefähr 3,8:1 für die erste Zone (16) zu der zweiten Zone (18) beträgt.

3. Elastomerer Artikel nach Anspruch 1, wobei die erste Zone (16) eine Oberfläche aufweist, welche ein unbedecktes elastomeres Material ist oder mit einem klebrigmachenden Material beschichtet ist.

4. Elastomerer Artikel nach einem der vorangehenden Ansprüche, wobei die erste Region (16) Teil einer Oberfläche ist, die dafür eingerichtet ist, das Rutschen des Artikels zu verhindern, wenn sie entweder mit menschlicher Haut oder mit einem Gewebematerial in Kontakt ist.

5. Elastomerer Artikel nach einem der vorangehenden Ansprüche, wobei der Artikel ein Handschuh (10) ist.

6. Elastomerer Artikel nach einem der vorangehenden Ansprüche, wobei die erste Region (16) einen Teil einer Handschuhmanschette bildet und die zweite Region (18) wenigstens einen Teil eines auf die Hand anzuziehenden Abschnitts des Handschuhs (10) bildet.

7. Elastomerer Artikel nach einem der vorangehenden Ansprüche, wobei die zweite Region (18) entweder eine wesentlich größere oder ungefähr die gleiche Oberfläche aufweist wie die erste Region (16) und wenigstens eine teilweise Beschichtung aus einer Anziehmaterialschicht aufweist.

8. Elastomerer Artikel nach einem der vorangehenden Ansprüche, wobei die innere Oberfläche (12) einem Oxidationsmittel ausgesetzt ist.

9. Handschuh nach einem der vorangehenden Ansprüche, wobei die innere Oberfläche (12) einer teilweisen Halogenierung ausgesetzt ist, so dass die erste Region nicht chloriert ist und die zweite Region chloriert ist.

10. Verfahren zum Herstellen eines elastomeren Artikels mit einer Oberfläche mit wenigstens zwei Regionen mit unterschiedlichen Reibungskoeffizienten, wobei das Verfahren umfasst: das Bereitstellen eines Latexsubstrats aus Naturkautschuk oder synthetischem Polymer auf einer Form; das Unterwerfen des Latexsubstrats einem ersten Auslaugen mit Wasser; das Aufbringen einer wässrigen AlSO₄-haltigen Lösung auf eine Oberfläche des Latexsubstrats; das Aufbringen einer Anziehbeschichtung über einem Teil der AlSO₄-überzogenen Oberfläche, wobei eine Zone von ungefähr wenigstens 1 Zoll (-2,2 cm) von einem Rand am Ende des Latexsubstrats her nicht mit der Anziehbeschichtung beschichtet wird; das Trocknen und Härten der Anziehbeschichtung; das Unterwerfen des Latexsubstrats einem zweiten Auslaugen mit Wasser; das Aufbringen einer zweiten Schicht von AlSO₄ über der Anziehbeschichtung; und das Aussetzen des Latexsubstrats einer verdünnten Lösung eines Oxidationsmittels unter alkalischen Bedingungen von ungefähr ph ≥ 8.

11. Verfahren nach Anspruch 10, wobei der pH ungefähr 9-11 beträgt.

12. Verfahren nach Anspruch 10, wobei die Anziehbeschichtung ein Hydrogelmaterial einschließt.

13. Verfahren nach Anspruch 10, wobei das Oxidationsmittel eine Hypochlorit-Lösung (NaOCl-Lösung) ist.

14. Verfahren nach Anspruch 10, außerdem umfassend das Abziehen des Latexsubstrats von der Form, das Umdrehen des Latexsubstrats und das Aufbringen einer aus einer Siliconemulsion gebildeten Schmierschicht.

15. Verfahren nach Anspruch 10, wobei die Siliconemulsion entweder der Zone, welche keine Anziehbeschichtung aufweist, eine klebrigmachende Wirkung verleiht oder der Anziehbeschichtung eine glatt bzw. glitschig machende Wirkung verleiht.

16. Verfahren nach Anspruch 10, außerdem umfassend das Behandeln des Latexsubstrats mit einer ersten und zweiten aufeinanderfolgenden Spülung mit Wasser und einem chlorhaltigen Wasser mit ∼30 - 75 ppm vol., und einer verdünnten salzsauren Lösung (HCl-Lösung) von ungefähr 0,1-0,85 Volumenprozent, um jegliches verbliebene pulverförmige Trennmittel nach den ersten und zweiten Wasserspülungen zu entfernen.

17. Verfahren nach Anspruch 10, außerdem umfassend das Aussetzen des Latexsubstrats einem Medium, das eine Kombination von HCl + Chlor + Siliconemulsion einschließt, in einem einzigen Verfahrensschritt, um eine klebrige Oberfläche auf einer nicht mit Anziehmaterial beschichteten Oberfläche und eine glatte Oberfläche auf einer mit Anziehmaterial beschichteten Oberfläche zu erzielen.

18. Verfahren nach Anspruch 10, außerdem umfassend das Aufbringen einer weiteren Schicht der Siliconemulsionsbehandlung.

19. Verfahren nach Anspruch 10, wobei der elastomere Artikel wenigstens eine erste und eine zweite Zone auf einer inneren Oberfläche aufweist, wobei die erste Zone einen relativ höheren Reibungskoeffizienten, entsprechend einem mittleren Verhältnis im Bereich von ungefähr 1,4:1 bis ungefähr 3,5:1, aufweist.

20. Verfahren nach Anspruch 19, wobei das mittlere Verhältnis des Reibungskoeffizienten zwischen der ersten und zweiten Region ungefähr 1,8:1 bis ungefähr 3,0:1 beträgt.

## Revendications

1. Article souple comprenant : un corps substrat (12) largement formé d'un matériau élastomère, ledit corps substrat (12) ayant une première surface (14) et une seconde surface ; ladite première surface est différentiée en au moins une première et une seconde zones (16, 18), chaque zone (16, 18) ayant un coefficient de friction, de telle sorte que le coefficient de friction de ladite première zone (16) est supérieur au coefficient de ladite seconde zone (18), selon un rapport moyen compris dans la gamme allant d'environ 1,2:1 à environ 4,5:1, ladite première zone (16) étant dépourvue de couche d'enfilage, et ladite seconde zone (18) étant pourvue de ladite couche d'enfilage.

2. Article élastomère selon la revendication 1, dans lequel ledit rapport moyen de coefficient de friction est compris entre environ 1,4:1 et environ 3,8:1 entre ladite première zone (16) et ladite seconde zone (18).

3. Article élastomère selon la revendication 1, dans lequel ladite première zone (16) a une surface qui est en un matériau élastomère nu ou enduit d'un matériau rendant pégueux.

4. Article élastomère selon l'une quelconque des revendications précédentes, dans lequel ladite première région (16) fait partie d'une surface adaptée à empêcher le glissement dudit article lorsqu'il est en contact soit avec la peau humaine, soit avec une étoffe.

5. Article élastomère selon l'une quelconque des revendications précédentes, ledit article étant un gant (10).

6. Article élastomère selon l'une quelconque des revendications précédentes, dans lequel ladite première région (16) fait partie du poignet d'un gant et ladite seconde région (18) fait au moins partie d'une portion d'enfilage, sur la main, dudit gant (10).

7. Article élastomère selon l'une quelconque des revendications précédentes, dans lequel ladite seconde région (18) est sensiblement plus grande ou approximativement égale en superficie à ladite première région (16), et elle est au moins partiellement revêtue d'une couche de matériau d'enfilage.

8. Article élastomère selon l'une quelconque des revendications précédentes, dans lequel ladite surface intérieure (12) est soumise à un agent oxydant.

9. Gant selon l'une quelconque des revendications précédentes, dans lequel ladite surface intérieure (12) est soumise à une halogénation partielle, de telle sorte que ladite première région est non-chlorée et ladite seconde région est chlorée.

10. Procédé de fabrication d'un article élastomère ayant une surface présentant au moins deux régions ayant des coefficients de friction différenciés, le procédé comprenant : la mise en place d'un substrat en latex de caoutchouc naturel ou de polymère synthétique sur un moule ; le fait de soumettre ledit substrat en latex à une première lixiviation à l'eau ; l'application d'une solution aqueuse contenant. AlSO₄ à une surface dudit substrat en latex ; l'application d'un revêtement d'enfilage sur une partie de ladite surface à enduction AlSO₄; le fait de laisser dépourvue du revêtement d'enfilage une zone d'au moins 1 pouce (-2,2 cm) depuis un bord terminal dudit substrat en latex ; le séchage et le durcissement de ladite couche d'enfilage ; le fait de soumettre ledit substrat en latex à une seconde lixiviation à l'eau ; l'application d'une seconde couche de AlSO₄ sur ledit revêtement d'enfilage ; et l'exposition dudit substrat en latex à une solution diluée d'un agent oxydant dans des conditions alcalines d'environ pH ≥ 8.

11. Procédé selon la revendication 10, dans lequel ledit pH est d'environ 9-11.

12. Procédé selon la revendication 10, dans lequel ledit revêtement d'enfilage inclut un matériau hydrogel.

13. Procédé selon la revendication 10, dans lequel ledit agent oxydant est une solution d'hypochlorite (NaOCl).

14. Procédé selon la revendication 10, comprenant, en outre, la séparation du substrat en latex d'avec le moule, le fait de retourner ledit substrat en latex et l'application d'une couche lubrifiante formée à partir d'une émulsion de silicone.

15. Procédé selon la revendication 10, dans lequel ladite émulsion de silicone confère soit un effet de pégosité à ladite zone dépourvue dudit revêtement d'enfilage soit un effet soyeux audit revêtement d'enfilage.

16. Procédé selon la revendication 10, comprenant, en outre, le fait de soumettre ledit substrat en latex à un premier et à un second rinçages consécutifs avec de l'eau, et de l'eau contenant du chlore à raison de ∼30-75 ppm en volume, et une solution chlorhydrique (HCl) diluée à environ 0,1-0,85 % en volume pour éliminer tout agent de démoulage pulvérulent résiduel après lesdits premier et second rinçages à l'eau.

17. Procédé selon la revendication 10, comprenant, en outre, le fait de soumettre ledit substrat en latex à un milieu incluant une combinaison de HCl + chlore + émulsion de silicone dans un procédé en une étape pour rendre pégueuse une surface sur une surface non revêtue d'un matériau d'enfilage et soyeuse une surface sur une surface revêtue d'un matériau d'enfilage.

18. Procédé selon la revendication 10, comprenant, en outre, l'application d'une autre couche d'émulsion de silicone comme traitement.

19. Procédé selon la revendication 10, dans lequel ledit article élastomère a au moins une première et une seconde zones sur une surface intérieure, ladite première zone ayant un coefficient de friction relativement supérieur, selon un rapport moyen d'environ 1,4:1 à environ 3,5:1.

20. Procédé selon la revendication 19, dans lequel ledit rapport moyen de coefficient de friction entre les première et seconde régions est d'environ 1,8:1 à environ 3,0:1.
